(19) Europäisches Patentamt
European Patent Office
Office européen des brevets

(11) Publication number: 0 033 663
A1

(12) # EUROPEAN PATENT APPLICATION

(21) Application number: 81300450.4

(22) Date of filing: 03.02.81

(51) Int. Cl.³: C 07 D 401/14
C 08 K 5/34, C 08 L 23/00
//(C07D401/14, 211/58, 233/02)

(30) Priority: 04.02.80 US 117878

(43) Date of publication of application: 12.08.81 Bulletin 81/32

(84) Designated Contracting States: BE CH DE FR GB IT LI NL SE

(71) Applicant: CARSTAB CORPORATION
West Street
Reading Ohio 45215(US)

(72) Inventor: Molt, Kenneth R.
5021 Cooper Road
Cincinnati Ohio 45242(US)

(74) Representative: Hose, Cyril Gustav Bidwell et al,
Baron & Warren 16 Kensington Square
London W8 5HL(GB)

(54) 2,2,6,6-Tetramethyl-4-piperidyl - substituted heterocyclic ring compounds, a method for their preparation and polymers containing the same.

(57) Novel 2,2,6,6-tetramethyl-4-piperidyl substituted nitrogen heterocyclic ring compounds containing two non-adjacent nuclear nitrogen atoms are provided which are useful as stabilisers for organic polymers. Organic polymers containing the 2,2,6,6-tetramethyl-4-piperidyl substituted nitrogen heterocyclic ring compounds dispersed therein exhibit improved resistance to deterioration induced by light, by heat or both. The 2,2,6,6-tetramethyl-4-piperidyl substituted nitrogen heterocyclic ring compounds may be prepared by a method comprising reacting a 2,2,6,6-tetramethyl-4-piperidyl substituted 1,2-aliphatic diamine with a monoaldehyde or dialdehyde.

EP 0 033 663 A1

"2,2,6,6-TETRAMETHYL-4-PIPERIDYL - SUBSTITUTED HETEROCYCLIC RING COMPOUNDS, A METHOD FOR THEIR PREPARATION AND POLYMERS CONTAINING THE SAME"

This invention relates to 2,2,6,6-tetramethyl-4-piperidyl - substituted heterocyclic ring compounds and to a method for their preparation and to stabilised polymer compositions containing such compounds. The invention relates to a method for the stabilisation of polymers wherein the said 2,2,6,6-tetramethyl-4-piperidyl - substituted heterocyclic ring compounds are admixed to the polymer.

That synthetic, and even many natural, organic polymers require stabilisation against the action of light, particularly ultraviolet light, and heat induced discoloration and deterioration of physical properties is well known. Organic polymers subjected to outdoor exposure often discolor and show a loss of physical properties as a result of the exposure to a number of degradation inducing factors (e.g. ultraviolet light, heat, water and oxygen). Outdoor use of organic polymers not only subjects the polymers to the action of the ultraviolet light of the sun but also the heat of the sun. This outdoor exposure to ultraviolet light and heat takes place in the presence of oxygen in the air. Thus, there can exist various combinations of factors working to degrade the organic polymer. On the other hand there exist other uses of organic polymers which result in their exposure to heat and oxygen in the absence of ultraviolet light, or ultraviolet light and oxygen in the absence of heat or even heat in the absence of oxygen. Thus, many organic polymers require protections against deterioration induced by a variety of environmental conditions. Factors such as heat and oxygen are not only present during the commercial use of organic polymers but are also present during the processing of the polymer into finished articles of commerce. Stabilisation of organic polymers against discoloration and loss of physical properties during processing into finished products is also required.

In view of the numerous applications and large scale use of organic polymers, it is very important to stabilise such

organic polymers against the deteriorating effects of environmental factors during processing and use. To protect many organic polymers against undesirable changes in physical properties and discolouration during processing and use, various stabilisers and stabiliser mixtures have been added to the organic polymers. Included among such stabilizers are, for example, the well known metal salts, organometallic compounds, phenols, hindered phenols, substituted and un substituted benzophenones, salicylates, mercaptans, expoxides and benzothiazoles. More recently hindered amine stabilisers having tetraalkyl substituted piperidino groups have been proposed as stabilisers. Although a large number of stabiliser compounds and stabiliser combinations, including the hindered amine type of stabilisers have been proposed and some of them used, there continues the search for better stabilisers to overcome the deficiencies of known stabilisers. Among these deficiencies there are, for example, included low stabilising efficiency, high cost, stabiliser instability, initial coloration of the polymer, volatility and odour. More effective, stable, easy to use and lower cost stabilisers are always sought.

It is, therefore, an object of this invention to provide a 2,2,6,6-tetramethyl-4-piperidyl substituted heterocyclic ring compound. Another object is to provide as a highly effective stabiliser for organic polymers a 2,2,6,6-tetramethyl-4-piperidyl substituted heterocyclic ring compound. A further object is to provide highly stable organic polymer compositions comprising an organic polymer and a 2,2,6,6-tetramethyl-4-piperidyl substituted heterocyclic ring compound. A further object is to provide a method for preparing the 2,2,6,6-tetramethyl-4-piperidyl substituted heterocyclic ring compounds of this invention.

In accordance with this invention there is provided 1) a compound having one or two bis(2,2,6,6-tetramethyl-4-piperidyl) substituted heterocyclic rings in which said

heterocyclic ring has five ring atoms, two of said ring atoms being non-adjacent nitrogen atoms whilst three are carbon atoms there being a 2,2,6,6-tetramethyl-4-piperidyl group bonded to each nitrogen atom of the heterocyclic ring (which compound is hereinafter called the hindered amine compound) and 2) a stabilised polymer composition comprising an organic polymer normally susceptible to light and/or heat induced degradation together with the above described hindered amine compound according to this invention. Further, this invention contemplates a method of stabilizing an organic polymer normally susceptible to light and/or heat induced degradation comprising the addition to said polymer of the above described hindered amine compound as well as a method for preparing the above described hindered amine compound comprising reacting a compound having the following general formula

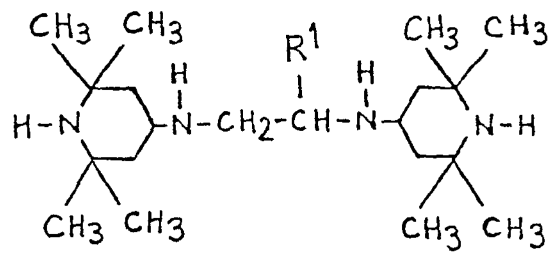

with an aldehyde having the general formula

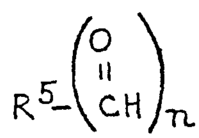

and, if desired, reacting the resulting product with a halide having the general formula $R^4Y$ wherein

$R^1$ is hydrogen or methyl,

$R^4$ is $C_1$ to $C_4$ alkyl, $C_7$ to $C_{18}$ aralkyl or $C_2$ to $C_8$ alkenyl,

$R^5$ is a straight or branched aliphatic, cycloaliphatic or substituted or unsubstituted aromatic group having a valence equal to n,

n is 1 or 2 and

Y is a halogen atom.

There has now been discovered a hindered amine compound,

as described herein, which can be used to stabilise organic polymers against light and/or heat induced degradation without many of the disadvantages of the prior art compounds used for the same purpose. The hindered amine compounds described herein in accordance with this invention have low volatility, are essentially non-migrating in an organic polymer and are readily compatible with organic polymers. Low volatility is an important feature especially during the incorporation of the stabiliser compound into the organic polymer and subsequent processing of the organic polymer into finished articles of commerce. Such low volatility reduces or prevents significant odours and contamination of the work place atmosphere as well as reducing or preventing undesirable losses through volatilisation of the stabiliser compound during processing. The non-migrating feature is advantageous for obtaining uniform distribution of the stabiliser throughout the organic polymer. High compatibility of the stabiliser with the polymer improves the mixing of the stabiliser into the polymer to achieve a uniform distribution of the stabiliser in the polymer. In addition to being useful as a stabiliser for organic polymers, it is contemplated that the hindered amine compound according to this invention may have such other uses as an insecticide, a fungicide, an anticorrosion agent, a bacteriocide and an antiviral agent.

In accordance with this invention there is provided a hindered amine compound, useful for stabilising organic polymers against light and/or heat induced degradation, having the general formula

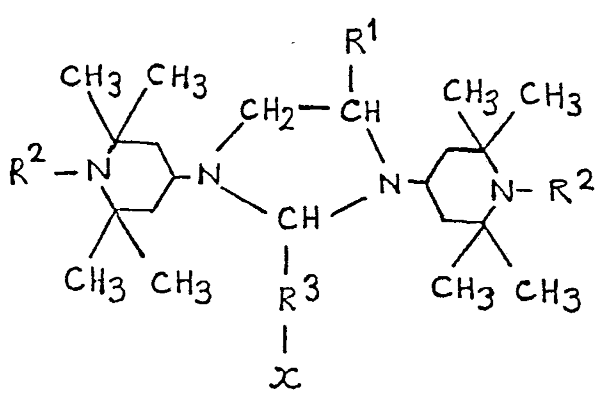

(I)

wherein

$R^1$ is hydrogen or methyl;

$R^2$ is hydrogen, alkyl having 1 to 8 carbon atoms, alkenyl having 2 to 8 carbon atoms or aralkyl having 7 to 18 carbon atoms;

$R^3$ is a straight or branched chain alkylene, straight or branched chain alkenylene, unsubstituted or substituted arylene or cycloalkylene group and

X is hydrogen or a group having the general formula

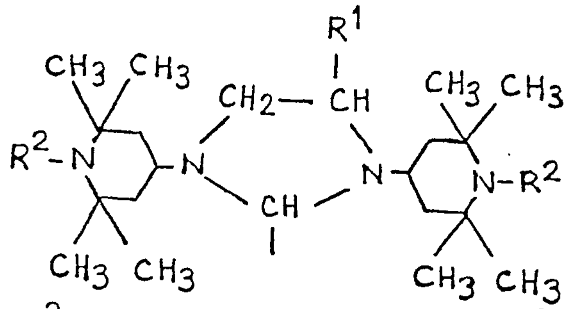

(II)

where $R^1$ and $R^2$ are as previously defined above. Further, there is provided in accordance with this invention an organic polymer composition comprising an organic polymer normally susceptible to light and/or heat induced degradation and a hindered amine compound according to formula (I). In addition, in accordance with this invention there is provided a novel method for making the hindered amine compound according to formula (I) characterised by reacting a compound having the following general formula

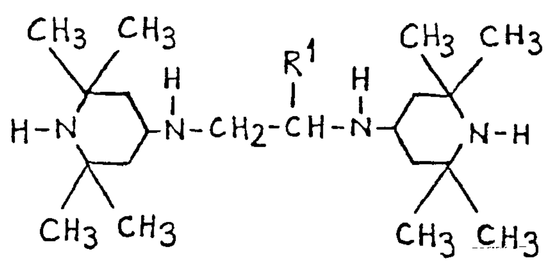

(III)

with an aldehyde having the general formula $R^5\left(\overset{O}{\overset{\|}{C}}H\right)_n$ and, if desired, reacting the resulting product with a halide having the general formula $R^4Y$ wherein

$R^1$ is as defined above for formula (I)

$R^4$ is alkyl having 1 to 8 carbon atoms, alkenyl having 2 to 8 carbon atoms or aralkyl having 7 to 18 carbon

atoms;

$R^5$ when n is 2, is a straight or branched chain alkylene, a straight or branched chain alkenylene, an unsubstituted or substituted arylene or cycloalkylene group; or

$R^5$ when n is 1, is a branched or straight chain alkyl, a straight or branched chain alkenyl, an unsubstituted or substituted aryl or a cycloalkyl group;

Y represents bromine, chlorine or iodine and

n is 1 or 2

A method for stabilising an organic polymer is also provided in accordance with this invention characterised by admixing with an organic polymer, normally susceptible to light and/or heat induced degradation, a hindered amine compound according to general formula (I) above.

Various embodiments of the hindered amine compound of this invention may be practised, including, but not limited to, the hindered amine compound according to formula (I) wherein $R^3$ is 1) a straight or branched chain alkylene group, preferably having 1 to 14 carbon atoms, 2) a straight or branched chain alkenylene group, preferably having 2 to 14 carbon atoms and one to three carbon to carbon double bonds, 3) a substituted or unsubstituted arylene group preferably alkyl substituted or unsubstituted phenylene or 4) a cycloalkylene group, preferably having six carbon atoms in the ring and wherein A) $R^1$ is H, $R^2$ is H and X is H; B) $R^1$ is H, $R^2$ is H and X is according to formula (II); C) $R^1$ is methyl, $R^2$ is H and X is H; D) $R^1$ is methyl, $R^2$ is H and X is according to formula (II); E) $R^1$ is H, $R^2$ is $C_1$ to $C_8$ alkyl (preferably $C_1$ to $C_4$ alkyl) and X is according to formula (II); G) $R^1$ is methyl, $R^2$ is $C_1$ to $C_8$ (preferably $C_1$ to $C_4$) alkyl and X is H; H) $R^1$ is methyl, $R^2$ is $C_1$ to $C_8$ (preferably $C_1$ to $C_4$) alkyl and X is according to formula (II); J) $R^1$ is H, $R^2$ is $C_7$ to $C_{18}$ (preferably $C_7$ to $C_{12}$) aralkyl and X is H; K) $R^1$ is H, $R^2$ is $C_7$ to $C_{18}$ (preferably $C_7$ to $C_{12}$) aralkyl and X is according to formula (II); L) $R^1$ is methyl, $R^2$ is $C_7$ to $C_{18}$ (preferably $C_7$ to $C_{12}$) aralkyl and X is H; M) $R^1$ is

methyl, $R^2$ is $C_7$ to $C_{18}$ (preferably $C_7$ to $C_{12}$ aralkyl and X is according to formula (II); N) $R^1$ is H, $R^2$ is $C_2$ to $C_8$ (preferably $C_2$ to $C_6$) alkenyl and X is H; P) $R^1$ is H, $R^2$ is $C_2$ to $C_8$ (preferably $C_2$ to $C_6$) alkenyl and X is according to formula (II); Q) $R^1$ is methyl, $R^2$ is $C_2$ to $C_8$ (preferably $C_2$ to $C_6$) alkenyl and X is H or S) $R^1$ is methyl, $R^2$ is $C_2$ to $C_8$ (preferably $C_2$ to $C_6$) alkenyl and X is according to formula (II).

Preferably the hindered amine compound according to this invention is a hindered amine compound according to formula (I) wherein $R^1$ is hydrogen, $R^2$ is hydrogen, methyl or benzyl, $R^3$ is a $C_2$ to $C_{12}$ straight or branched chain alkylene group of phenylene group and X is hydrogen or a group having general formula (II). Most preferably, the compound of this invention is a compound according to formula (I) wherein $R^1$ is hydrogen, $R^2$ is hydrogen, $R^3$ is a $C_2$ to $C_{12}$ straight or branched chain alkylene group or phenylene group and X is hydrogen or a group having general formula (II).

When $R^2$ is $C_1$ to $C_8$ alkyl the alkyl group can be a straight or branched chain alkyl group having from 1 to 8, preferably 1 to 4, carbon atoms. Such an alkyl group can, for example, be a methyl, ethyl, propyl, isopropyl, butyl, tertiary butyl, hexyl or 2 ethyl hexyl group. The $R^2$ may be a $C_7$ to $C_{18}$, preferably $C_7$ to $C_{12}$, aralkyl group. Such aralkyl group may be unsubstituted or have up to 3 substituents, preferably alkyl or alkoxy substituents having 1 to 4 carbon atoms, on the aryl moiety. Examples of such an aralkyl group include benzyl, phenethyl, phenbutyl, phenisopropyl, o-methylbenzyl, m-methylbenzyl, p-methylbenzyl, p-tertiary butylbenzyl, 3,5-dimethylbenzyl and methoxybenzyl. When $R^2$ represents a $C_2$ to $C_8$, preferably $C_2$ to $C_6$, alkenyl group the alkenyl group may be a straight or branched chain alkenyl group having 1 to 2, preferably 1, carbon to carbon double bond. For example, such an alkenyl group includes vinyl,

allyl, methallyl, 1-butenyl, 1-pentenyl, 1-hexenyl, 3-hexenyl and 1,3 pentadienyl.

In one aspect of the hindered amine compound of this invention according to formula (I) $R^3$ can be a straight or branched chain alkylene group, preferably having from 1 to 14 carbon atoms. Such alkylene groups, for example, include but are not limited to ethylene, propylene, tetramethylene, hexamethylene, octamethylene, decamethylene, tetradecamethylene, ethylethylene, 4-propyl-1,5-pentylene and 2-ethyl-1,6-hexylene. In another aspect of the hindered amine compound of this invention according to formula (I) $R^3$ may be a straight or branched chain alkenylene, preferably having 2 to 14 carbon atoms and from 1 to 3 (more preferably 1), carbon to carbon double bond, for example, vinylene, 4-propyl-2-pentenylene, 2-butenylene and 2-methyl-4-hexenylene. In accordance with formula (I) $R^3$ can be a substituted or unsubstituted arylene group. Such arylene group is preferably an unsubstituted arylene group having a six carbon atom ring (e.g. 1,4-phenylene, 1,3-phenylene and 1,2-phenylene). However, other arylene groups having a 5 carbon atom ring or a 7 carbon atom ring may also be used as $R^3$ in the practice of the hindered amine compound of this invention according to formula (I). When $R^3$ is a cycloalkylene group such group preferably contains 5 to 7, more preferably 6, carbon atoms in the cycloalkylene ring (e.g. 1,4-cyclohexylene). In the context of the hindered amine compound of this invention according to formula (I), the term alkylene, alkenylene, arylene and cyclohexylene are meant to identify divalent organic radicals.

The hindered amine compound of this invention, more preferably the compound according to formula (I) may be prepared by a method which constitutes one aspect of this invention. Thus, the hindered amine compound of this invention according to formula (I) may be prepared by a method which is characterised by reacting a compound having the general formula

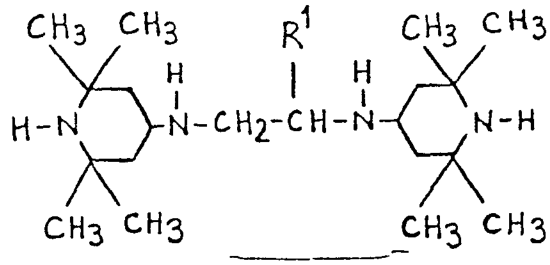

(III)

with an aldehyde having the general formal $R^5\left(\overset{O}{\overset{\|}{C}}H\right)_n$ and optionally reacting the resulting product of said reaction with a halide having the general formula $R^4Y$ wherein $R^1$, $R^4$, $R^5$, Y and n are defined above.

The compound having the general formula III may be prepared by methods known in the art such as, for example, reacting triacetone amine with ethylene diamine and reducing the resulting product in accordance with well known methods. More detailed descriptions of the preparation of the hindered amine compounds of this invention by the method forming an aspect of this invention are given in the examples contained herein. It will be recognised from the description herein that 1) the $R^4$ radical of the compound having the formula $R^4Y$ corresponds to the $R^2$ radical of the compound having the general formula (I) and 2) the $R^5$ radical of the aldehyde having the general formula $R^5\left(\overset{O}{\overset{\|}{C}}H\right)_n$ corresponds to the $R^3$ radical of the compound having formula (I). As examples of aldehydes having the general formula $R^5\left(\overset{O}{\overset{\|}{C}}H\right)_n$ there are included acetaldehyde, butyraldehyde, caproaldehyde, dodecylaldehyde, 2-ethyl-butyraldehyde, acrolein, transcinnamaldehyde, 2,4-heptadienal, 2,4-hexadienal, benzaldehyde, methacrolein, phenyl acetaldehyde, 2-phenyl propionaldehyde, p-anisaldehyde, m-anisaldehyde, 2,4-dimethyl benzaldehyde, 2,3-dimethylbenzaldehyde, 2,4-dimethoxybenzaldehyde, isophthaldehyde, mesitaldehyde, o-phthalicdicarboxaldehyde, terephthalicdicarboxaldehyde and glutaraldehyde. Halides having the formula $R^4Y$, include methyl chloride, ethyl bromide, hexyl iodide, octyl chloride, allyl choride, 1-bromo-1-propene,

benzyl chloride and (2-chloroethyl)-benzene.

There is provided in accordance with this invention a polymer composition having improved resistance to deterioration, induced by light, by heat or by a combination of light and heat, comprising an organic polymer normally susceptible to deterioration induced by light, by heat or a combination of light and heat and characterised by having disposed therethrough a stabilisingly effective amount of a compound having one or two bis(2,2,6,6-tetra-methyl-4-piperidyl) substituted heterocyclic rings in which said heterocyclic ring has five ring atoms, two of which are non-adjacent nitrogen atoms and three of which are carbon atoms and having a 2,2,6,6-tetramethyl-4-piperidyl group bonded to each nitrogen atom of the heterocyclic ring. Further, in accordance with this invention there is provided an organic polymer composition comprising an organic polymer normally susceptible to deterioration induced by light, by heat or by both and dispersed therethrough a stabilisingly effective amount of a hindered amine compound according to formula (1).

As organic polymers, normally susceptible to deterioration induced by light, by heat or by both, usable in the practice of this invention, there are included both natural and synthetic organic polymers. These natural and synthetic organic polymers, for example, include but not limited to a) homopolymers and copolymers of hydrocarbon monomers having one or two olefinic double bonds (e.g. polyethylene, polypropylene, polybutene-1, polyisobutene, polymethylbutene-1, polymethylpentene-1, polyisoprene, polybutadiene, polystyrene, acrylonitrile-butadiene-styrene, terpolymer, ethylenepropylene copolymers, propylene-butene-1 copolymers, ethylene-butene-1 copolymers propylene-isobutene copolymers and styrenebutadiene copolymers), b) terpolymers (e.g. terpolymers of ethylene and propylene with diene monomers such as cyclopentadiene, ethylidenenorborene, hexadiene and pentadiene-1,4), c) polymerised acrylates and methacrylates (e.g. polymethyl

acrylate, polymethyl methacrylate, polybutyl acrylate, polyhexyl acrylate and butyl acrylate-methyl methacrylate copolymer?, d) derivatives of cellulose (e.g. cellulose acetate, cellulose butyrate, cellulose acetate propionate and cellulose acetate butyrate), e) polyurethanes, f) vinyl halide homopolymers, vinyl halide copolymers and polymer blends containing vinyl halide homopolymers or vinyl halide copolymers usable in the practice of this invention there, for example, may be used (1) polyvinyl chloride, polyvinylidene chloride, polyvinyl bromide, polyvinyl fluoride, polyvinylidene fluoride, (2) copolymers of vinyl chloride with a copolymerisable ethylenically unsaturated monomer such as vinylidene chloride, vinyl acetate, vinyl butyrate, vinyl benzoate, diethyl fumarate, diethyl maleate, other alkyl fumarates and maleates, vinyl propionate, methyl acrylate, 2-ethylexyl acrylate, butyl acrylate, ethyl acrylate, and other alkyl acrylates methyl methacrylate, ethyl methacrylate, butyl methacrylate, hydroxyethyl methacrylate and alkyl methacrylate, methyl alpha chloracrylate, styrene, vinyl ethers such as vinyl ethyl ether, vinyl chloroethyl ether, vinyl phenyl ether, vinyl ketones such as vinyl methyl ketone, vinyl phenyl ketone, 1 fluoro-1-chloroethylene, acrylonitrile, chloroacrylonitrile, allylidene diacetate, chloroallylidene diacetate, ethylene and propylene, and (3) polymer blends such as blends of polyvinylchloride and polyethylene, polyvinyl chloride and chlorinated polyethylene, polyvinyl chloride and polymethyl methacrylate, polyvinyl chloride and polybutyl methacrylate, polyvinyl chloride and polystyrene, polystyrene, polyvinyl chloride and acrylonitrile-butadiene-styrene copolymer and polyvinyl chloride and polyethylene and polymethyl methacrylate, g) polymerised non-halogenated vinyl compounds (e.g. polymerised vinyl acetate, vinyl propionate, vinyl butyrate, vinyl benzoate and copolymerised vinylidine compounds such as ethylene-vinyl acetate copolymers, vinyl acetate-vinyl butyrate copolymers and vinyl propionate-vinyl benzoate copolymers) and

h) natural polymers (e.g. cellulose and rubber). Mixtures of organic polymers containing at least one organic polymer normally susceptible to deterioration induced by light, by heat or by both light and heat can be used in the organic polymer compositions of this invention.

Preferably in the practice of the organic polymer compositions of this invention there are used synthetic organic polymers normally susceptible to deterioration induced by light, by heat or by both. More preferably there are used homopolymers and copolymers of olefinically unsaturated monomers and mixtures containing said homopolymers and copolymers. Most preferably there are used homopolymers and copolymers of ethylene and propylene and mixtures containing said homopolymers and copolymers.

In one particular aspect the organic polymer composition of this invention comprises a synthetic organic polymer normally susceptible to deterioration induced by light, by heat or by both and a stabilisingly effective amount of a hindered amine compound according to formula (I), more particularly a hindered amine compound according to formula (I) wherein $R^1$ is hydrogen, $R^2$ is hydrogen, methyl or benzyl, $R^3$ is a $C_2$ to $C_{12}$ straight or branched chain alkylene group or a phenylene group and X is hydrogen or a group according to general formula (II), most preferably a hindered amine compound according to formula (I) wherein $R^1$ is hydrogen, $R^2$ is hydrogen, $R^3$ is a $C_2$ to $C_{12}$ straight or branched chain alkylene group or a phenylene group and X is hydrogen or a group according to general formula (II). Another aspect of the organic polymer composition of this invention provides an organic polymer composition comprising a homopolymer or copolymer of an olefinically unsaturated monomer, more particularly ethylene and propylene, or a polymer mixture containing said homopolymer or copolymer and a stabilisingly effective amount of a hindered amine compound according to formula (I), more particularly a hindered amine compound according to general formula (I)

wherein $R^1$ is hydrogen, $R^2$ is hydrogen, methyl or benzyl, $R^3$ is a $C_2$ to $C_{12}$ straight or branched chain alkylene group or a phenylene group and X is a hydrogen or a group according to general formula (II), most preferably a hindered amine compound according to general formula (I) wherein $R^1$ is hydrogen, $R^2$ is hydrogen, $R^3$ is a $C_2$ to $C_{12}$ straight or branched chain alkylene group or a phenylene group and X is a hydrogen or a group according to general formula (II).

A still further aspect of the organic polymer composition of this invention constitutes an organic polymer composition comprising a homopolymer or copolymer of a vinyl halide monomer or a polymer mixture containing a homopolymer or copolymer or a vinyl halide monomer and a stabilising effective amount of a hindered amine compound according to general formula (I), more particularly, a hindered amine compound according to general formula (I) wherein $R^1$ is hydrogen, $R^2$ is hydrogen, methyl or benzyl, $R^3$ is a $C_2$ to $C_{12}$ straight or branched chain alkylene group or a phenylene group and X is hydrogen or a group according to general formula (II), most preferably a hindered amine compound according to general formula (I) wherein $R^1$ is hydrogen, $R^2$ is hydrogen, $R^3$ is a $C_2$ to $C_{12}$ straight or branched chain alkylene group or a phenylene group and X is hydrogen or a group according to formula (II).

In accordance with the preferred practice of the organic polymer composition of this invention there is provided an organic polymer composition comprising an organic polymer which is a homopolymer and a copolymer of ethylene and propylene and a polymer mixture containing said homopolymer or copolymer and dispersed therethrough a stabilisingly effective amount of a hindered amine compound according to formula (I) wherein $R^1$ is hydrogen, $R^2$ is hydrogen, $R^3$ is a $C_2$ to $C_{12}$ straight or branched chain alkylene group or a phenylene group and X is hydrogen or a group according to formula (II).

Various organic polymers, normally susceptible to

deterioration induced by light, by heat or by both, can be used in the organic polymer compositions of this invention. Such organic polymers can require widely different amounts of the compound having one or two bis(2,2,6,6-tetramethyl-4-piperidyl) substituted heterocyclic rings having five ring atoms, two of which are non-adjacent nitrogen atoms and three of which are carbon atoms and having a 2,2,6,6-tetramethyl-4-piperidyl group bonded to each nitrogen atom of the heterocyclic ring, preferably the hindered amine compound according to formula (I), for obtaining a stabilising effect and, therefore, the amount of the hindered amine compound or the hindered amine compound according to formula (I) to be used in the practice of the organic polymer composition of this invention can vary over a wide range. In the practice of the organic polymer composition of this invention, the hindered amine compound or the hindered amine compound according to formula (I) may be used in amount of from 0.001% to 10%, preferably 0.01% to 5%, more preferably 0.5% to 3% by weight based on the organic polymer. There may be used an amount of the hindered amine compound or the hindered amine compound according to formula (I) which is substantially greater than the amount needed to stabilise the organic polymer; however, such greater amount of the hindered amine compound or the hindered amine compound according to formula (I) is not needed and can even be wasteful and economically undesirable. It is contemplated that in the organic polymer composition of this invention any of the hindered amine compound or the hindered amine compound having the general formula (I) may be used in amounts in accordance with the above teachings.

The organic polymer compositions according to this invention may, in addition to the hindered amine compound or the hindered amine compound according to formula (I), contain conventional additives such as, for example, fillers, pigments, plasticisers, dyes, lubricants and stabilisers well

known in the art. Among the fillers, such materials as calcined clays, calcium carbonate and talcs may be used. Pigments well known in the art may be used including such materials as titanium dioxide, carbon black and iron oxide. Included among the well known plasticisers which may be used are the phthalates, sebacates, adipates, phosphates and fatty esters having between 16 and 150 carbon atoms in the molecule. Lubricants well known in the art which may be used include hydrocarbon waxes, stearyl stearate, cetyl palmitate and other ester waxes. Stabilisers which may be used include the well known ortho hydroxybenxophenones, hydroxybenzotriazoles, organotin carboxylates, organotin sulfides and organotin mercaptocarboxylic acid esters. Antioxidants which can be used include, for example 2,6-di-(-t-butyl-)-4-methyl phenol; 2,6-di(t-butyl)-4-decyloxy phenol and 2-t-butyl-4-octadecyloxy phenol.

Methods well known in the art for compounding plastic compositions for subsequent processing by techniques such as injection moulding, extrusion and the like may be used for the preparation of the organic polymer compositions of this invention. Such methods include dry blending with conventional mixers such as the well known Henschel blender, blending on a two roll or three roll mill and tumbling. The hindered amine compound or the hindered amine compound according to general formula (I) may be added to the organic polymer separately or in admixture with one or more conventional additives (e.g. plasticiser, antioxidant and lubricant). Where the hindered amine compound or the hindered amine compound according to formula (I) is a solid, it may for the sake of convenience be added (i.e. dissolved or dispersed) in a liquid carrier and the resulting admixture added to the organic polymer.

The organic polymer composition of this invention may advantageously be used to make articles of commerce such as, for example, seating, storage containers, toys and pipe.

Advantages such as, for example, improved processing

stability (i.e. improved resistance to break down and discoloration during processing) and greater durability of the moulded article (i.e. greater resistance of the moulded article to deterioration upon exposure to light, to heat or both are obtained from the organic polymer composition of this invention.

There is also provided in accordance with this invention a process for making a compound having one or two bis(2,2,6,6-tetramethyl-4-piperidyl) substituted heterocyclic rings in which said heterocyclic ring has five ring atoms, two of which are non-adjacent nitrogen atoms and three of which are carbon atoms, and having a 2,2,6,6-tetramethyl-4-piperidyl group bonded to each nitrogen atom of the heterocyclic ring characterised in that it comprises reacting a N,N$^1$-bis(2,2,6,6-tetramethyl-4-piperidyl) substituted 1,2-ethylene diamine or 1,2-propylene diamine with an aliphatic monoaldehyde or dialdehyde, a cycloaliphatic monoaldehyde or dialdehyde, an aromatic monaldehyde or dialdehyde, an arylaliphatic monoaldehyde or dialdehyde or an alkylaromatic monoaldehyde or dialdehyde and, if desired, reacting the resulting product of said reaction with an alkyl halide, an aralkyl halide or an alkenyl halide. Further, there is provided in accordance with this invention a process for making a hindered amine compound having the general formula

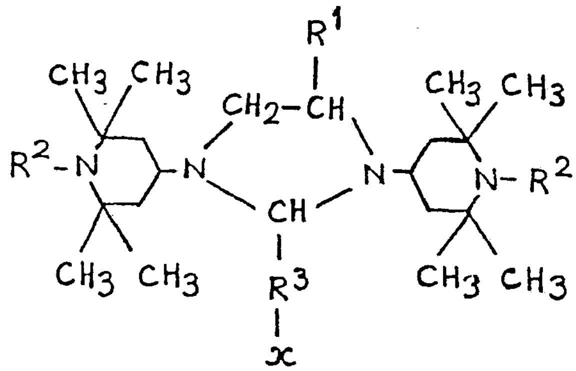

(I)

wherein

$R^1$ is hydrogen or methyl,

$R^2$ is hydrogen, $C_1$ to $C_8$ alky, $C_2$ to $C_8$ alkenyl or

$C_7$ to $C_{18}$ aralkyl,

$R^3$ is a straight or branched chain alkylene group, a straight or branched chain alkenylene group, a substituted or unsubstituted arylene group of a cycloalkylene group and

X is hydrogen or a group having the general formula

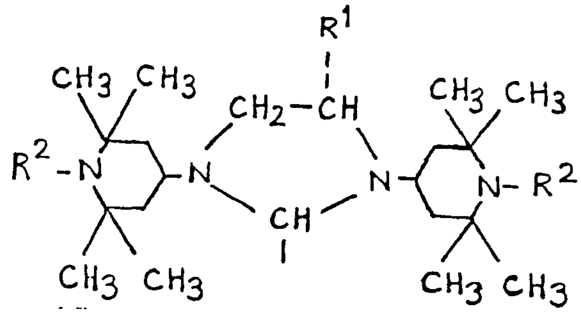

(II)

where $R^1$ and $R^2$ are as defined above, characterised in that it comprises reacting a compound having the general formula

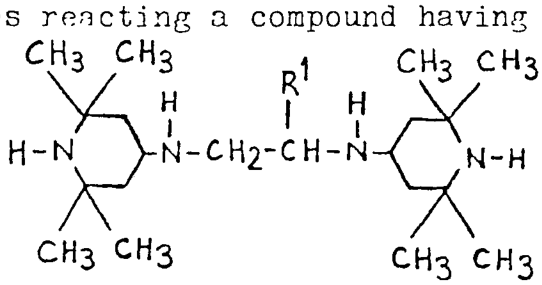

(III)

with an aldehyde having the formula $R^5\left(\overset{\overset{O}{\|}}{C}H\right)_n$ and, if desired, reacting the resulting product of said reaction with a halide having the formula $R^4Y$ wherein $R^1$ is hydrogen or methyl, $R^4$ is $C_1$ to $C_8$ alkyl group, a $C_2$ to $C_8$ alkenyl group or a $C_7$ to $C_{18}$ aralkyl group, $R^5$ is when n is 1, a branch or straight chain alkyl group, a straight or branched chain alkenyl group, a substituted or unsubstituted aryl group or a cycloalkyl group or when n is 2, a straight or branched chain alkylene group, a straight or branched chain alkenylene group, a substituted or unsubstituted arylene group or a cycloalkylene group, Y is bromine, chlorine or iodine and n is 1 or 2.

In accordance with one embodiment of the process of this invention, the optional reaction of halide having the general formula $R^4Y$ with the product of the reaction of a compound

according to the general formula (III) with an aldehyde having the general formula $R^5\left(\overset{O}{\overset{\parallel}{C}}H\right)_n$ is omitted. Another embodiment of the process of this invention for making a hindered amine compound according to general formula (I) comprises reacting a compound having the general formula (III), wherein $R^1$ is hydrogen or methyl, with an aldehyde having the general formula $R^5\left(\overset{O}{\overset{\parallel}{C}}H\right)_n$ wherein $R^5$ is a straight or branched chain alkyl group or substituted or unsubstituted aryl group (preferably a $C_1$ to $C_{12}$ straight or branched chain alkyl group or phenyl group) and n is 1. A further embodiment of the process of this invention for making a hindered amine compound according to general formula (I) comprising reacting a compound according to general formula (III), wherein $R^1$ is hydrogen or methyl, with an aldehyde having the general formula $R^5\left(\overset{O}{\overset{\parallel}{C}}H\right)_n$ wherein $R^5$ is a straight or branched chain alkylene group or a substituted or unsubstituted arylene group (preferably a $C_2$ to $C_{12}$ straight or branched chain alkylene group or a phenylene group) and n is 2. In another embodiment of the process there is provided a method for making a hindered amine compound according to general formula (I) characterised by reacting a compound according to general formula (III), wherein $R^1$ is hydrogen or methyl, with an aldehyde having the general formula $R^5\left(\overset{O}{\overset{\parallel}{C}}H\right)_n$ wherein $R^5$ is a straight or branched chain alkyl group or a substituted or unsubstituted aryl group, preferably a $C_1$ to $C_{12}$ straight or branched chain alkyl group or phenyl, and n is 1 and thereafter reacting the product with a halide having the general formula $R^4Y$ wherein $R^4$ is a $C_1$ to $C_8$ (preferably $C_1$ to $C_4$) alkyl group or a $C_7$ to $C_{18}$ (preferably $C_7$ to $C_9$) aralkyl group and Y is iodine or chlorine (preferably iodine). A still further embodiment of the process provides a method of making a hindered amine compound according to general formula (I) comprising reacting a compound having general formula (III),

wherein $R^1$ is hydrogen or methyl, with an aldehyde having the general formula

$R^5\left(\overset{\overset{O}{\|}}{C}H\right)_n$ wherein $R^5$ is a straight or branched chain alkylene group or a substituted or unsubstituted arylene group (preferably a $C_2$ to $C_{12}$ straight or branched chain alkylene group or a phenylene group) and n is 2 and thereafter reacting the product with a halide having the general formula $R^4Y$ wherein $R^4$ is a $C_1$ to $C_8$ (preferably $C_1$ to $C_4$) alkyl group or a $C_7$ to $C_{18}$ (preferably $C_7$ to $C_9$) aralkyl group and Y is iodine or chlorine (preferably iodine).

The preferred process of this invention is a method for preparing a hindered amine compound having general formula (I) characterised by reacting a compound having general formula (III) wherein $R^1$ is hydrogen, with an aldehyde having the general formula R

$R^5\left(\overset{\overset{O}{\|}}{C}H\right)_n$ wherein $R^5$ is, when n is 1, a straight or branched chain alkyl group, preferably having 1 to 12 carbon atoms or a substituted or unsubstituted aryl (preferably phenyl)

group, or when n is 2, a straight or branched chain alkylene group preferably having 2 to 12 carbon atoms of a substituted or unsubstituted arylene group, preferably phenylene and n is 1 or 2.

There may be used in the process of this invention an inert liquid medium, preferably an inert liquid hydrocarbon medium, for carrying out the reaction of the N,N$^1$-bis(2,2,6,6-tetramethyl-4-piperidyl) substituted 1,2-ethylene diamine or 1,2-propylene diamine (preferably a compound having general formula (III)) with a monoaldehyde or dialdehyde (preferably a monoaldehyde or dialdehyde having the general formula $R^5\left(\overset{O}{\overset{\|}{C}}H\right)_n$ as previously described herein) and if desired, reacting the product with a compound having the general formula $R^4Y$. This inert liquid medium, which is preferably a liquid hydrocarbon, may be a mixture of several organic liquids at least one of which may be non-hydrocarbon organic liquid which is insert to the reactants. Preferably there is at least one liquid hydrocarbon present in such mixture of several organic liquids. Although the use of elevated temperatures is generally preferred in the process such elevated temperatures will depend upon the nature and composition of the reactants as well as any reaction medium employed. It is, however, desirable to use a temperature high enough to remove by-product water formed during the reaction without decomposing the reactants and/or the hindered amine compound product. A temperature equal to the reflux temperature of the inert liquid medium may be used provided such reflux temperature is not a temperature causing decomposition of the reactants and/or the hindered amine compound product. Atmospheric pressure or a pressure above or below atmospheric pressure may be used in the process. Preferably atmospheric pressure is employed for carrying out the reaction to produce the hindered amine compound product in accordance with this invention.

In the process of this invention there may be used stoichiometric amounts of the reactants or in some circumstances there may advantageously be used amounts of a reactant in excess of the stoichiometric amount.

The hindered amine compound of this invention is useful in stabilising organic polymers (e.g. polyethylene and polypropylene) against deterioration (e.g. deterioration induced by light, by heat or both.

The following non-limiting examples and preparations further describe this invention and the process thereof. All amounts, ratios and percentages are by weight and all temperatures are in degrees Centigrade,

PREPARATION A

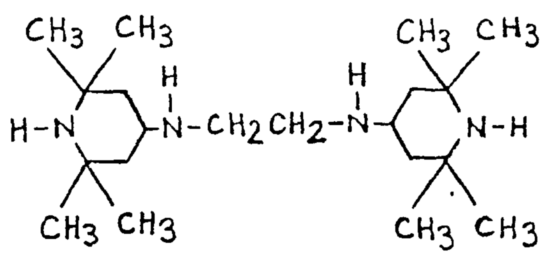

This is an example of the preparation of the above reactant which is used for the preparation of a hindered amine compound of this invention.

One mole of triacetone amine and 0.5 moles of ethylene diamine were dissolved in 200 grams of heptane and refluxed into a water trap for 1.0 hour. The heptane was removed by stripping to 110°C at 15 mm Hg. The residue was dissolved in 300 grams of isopropanol and added to a flask containing 1.0 mole of $NaBH_4$ in 300 grams of isopropanol. After reacting at 40-45°C for 10 hours, 200 grams of heptane was added, followed by 500 grams of water. The organic layer was stripped to 130°C at 15 mm to yield 166.0 grams of crude product. Recrystallisation of the product from heptane gave 133.0 grams (72%) of white crystals melting at 67-70°C. Analysis by GLC indicated 99.3% purity.

PREPARATION B

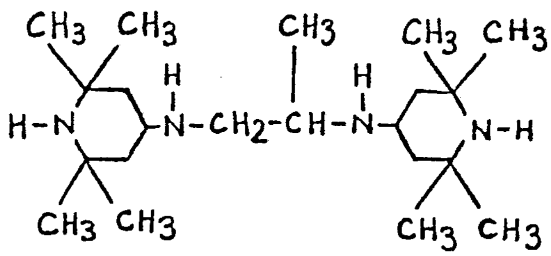

This illustrates the preparation of the above reactant which is used for the preparation of another hindered amine compound of this invention. The procedure of Preparation A followed except that 1,2-diaminopropane was used in place of ethylene diamine.

| | | |
|---|---|---|
| Yield | - | 91.6 grams |
| Appearance | - | White Crystals |
| Melting point | - | 49.51°C |

Example I:

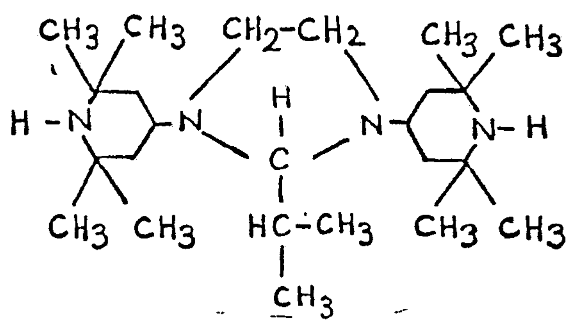

The above compound was prepared in the following manner.

One tenth mole of the compound of Preparation A and 0.3 moles of isobutyraldehyde were dissolved in 100 grams of toluene and refluxed for two hours using a water trap. The solvent was removed by stripping to 120°C at 15 mm and the crude residue recrystallised from 50 grams of heptane.

| | | |
|---|---|---|
| Yield | - | 29.6 grams (75.5%) |
| Melting Point | - | 92-96°C |
| Colour | - | White |
| GLC | - | Shows only one peak |
| IR & NMR | - | Consistent with the above structure |

Example II:

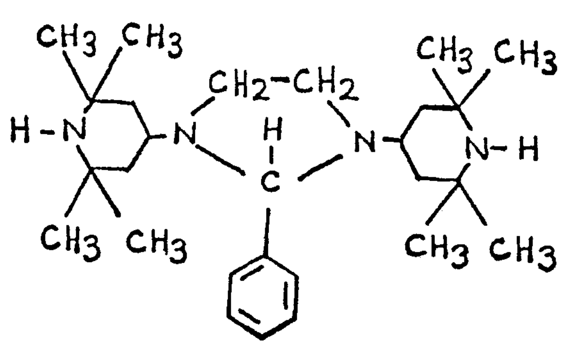

This compound was made by the method of Example I using benzaldehyde in place of isobutyraldehyde.

| | | |
|---|---|---|
| Yield | - | 59.6 grams (93.1%) |
| Melting Point | - | 87-91°C |
| GLC | - | Only one peak |
| IR & NMR | - | Consistent with the above structure |

Example III:

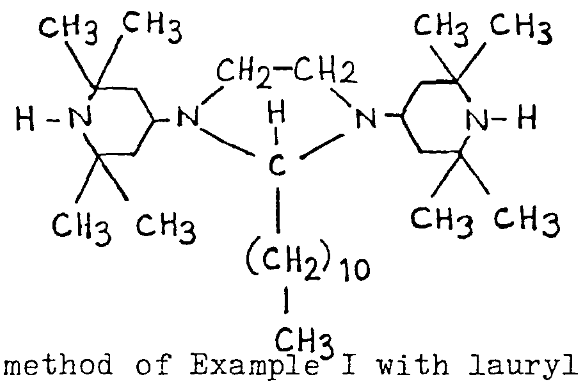

Using the method of Example I with lauryl aldehyde replacing isobutyraldehyde the above compound was obtained in nearly quantitative yield as a yellow oil. IR and NMR analyses were consistent with the above structure.

Example IV:

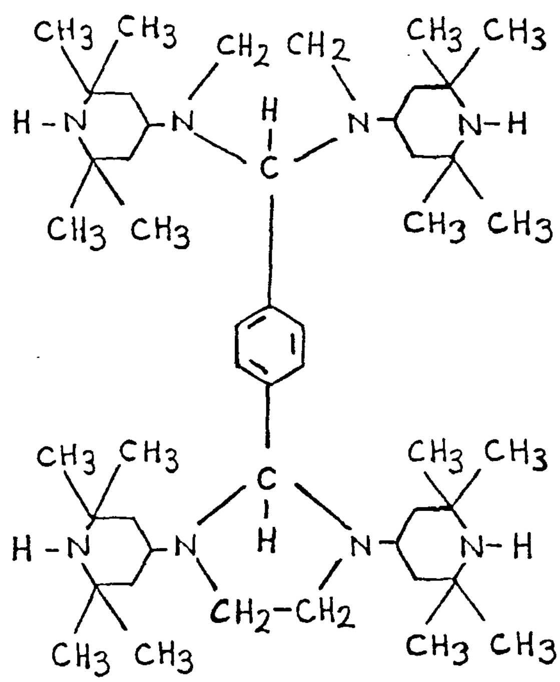

The above compound was prepared in the following manner.

One tenth mole of the compound of Preparation A and 0.05 mole of terephthaldehyde in 70 grams of heptane were refluxed for 15 hours using a water trap. The solution was cooled to 20°C and filtered to remove the product.

| | |
|---|---|
| Yield | - 37.6 grams (97.1%) |
| Melting Point | - 265-270°C |
| Colour | - White |
| IR & NMR | - Consistent with the above structure |

Example V:

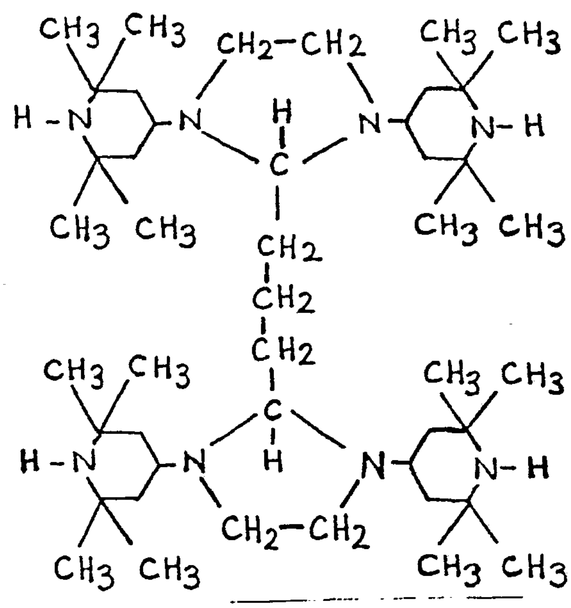

The above compound was prepared in the following manner.

One tenth mole of the compound of Preparation A and 0.05 mole of glutaraldehyde (49% in water) was mixed with 50.0 grams of xylene and 100 grams of dimethylacetamide. After refluxing using water trap for 3 hours the solvent was removed by stripping to 130°C/0.5 mm Hg. The crude product was obtained in 97% yield as a yellow glass. IR and NMR on crude product were consistent with the above structure.

Example VI:

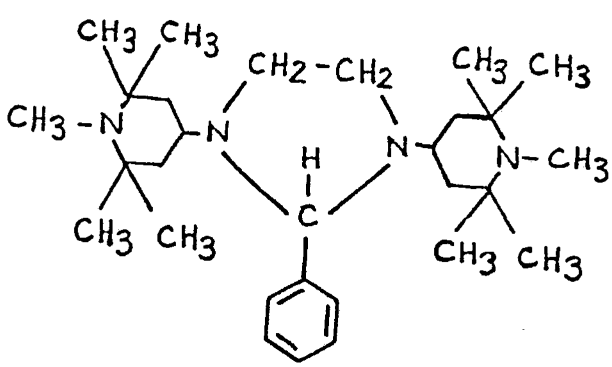

In preparing the above compound the following method was used. The compound of Example II (0.05 mole) was dissolved in 60 grams of isopropanol and reacted with 0.2 moles methyl iodide at 35-38°C for 3 hours. There was added 80 grams of toluene, 100 grams of water and then 0.20 moles of 50% NaOH. The toluene layer was stripped to 120°C at 15 mm to yield 12.0 grams of crude product as a yellow glass. IR and NMR spectra on the crude product was consistent with the above structure

Example VII:

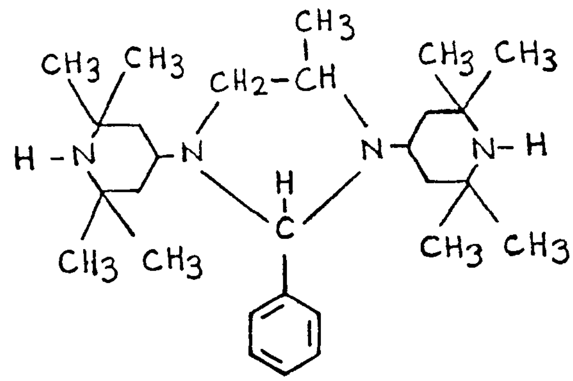

One tenth of the compound of Preparation B was mixed with 0.2 moles of benzaldehyde and 120 grams of toluene. After refluxing into a water trap for 2 hours the solvent was stripped off up to 140°C at 15 mm. The crude product was recrystallised from hexane.

| | |
|---|---|
| Yield | - 24.1 grams |
| Colour | - White |
| Melting Point | - 51.56°C |
| GLC | - Only one peak |
| IR & NMR | - Consistent with above structure |

Example VIII:

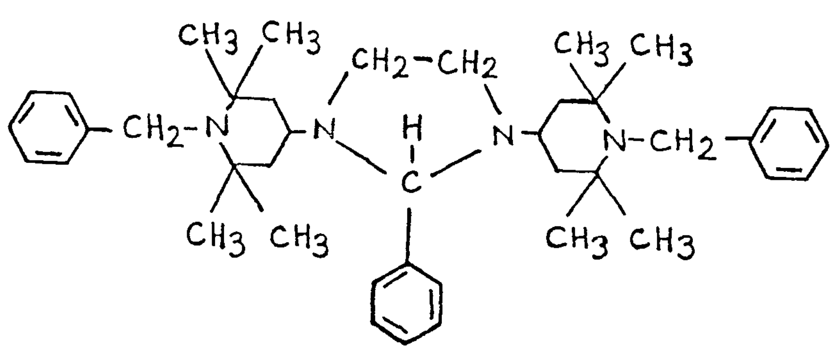

The compound of Example II (.05 moles) was dissolved in 80 gms of isopropanol and reacted with 0.3 moles of benzyl chloride at reflux for 18 hours. The reaction mixture was diluted with 100 gms of toluene and treated with 0.3 moles of dilute NaOH. The toluene layer was stripped to 120°C/0.5 mm to yield the crude product as a yellow glass. IR & NMR spectra in accord with the indicated structure.

Example IX:

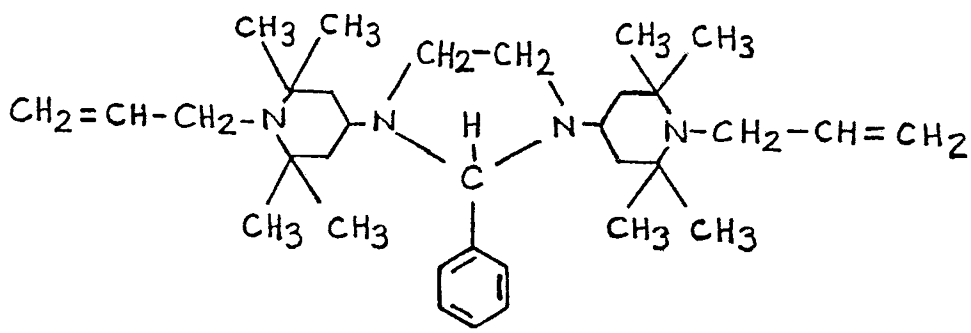

The compound of this example was made by the method of Example VIII by replacing the benzyl chloride with allyl chloride.

Yield - 14.0 gms

Form - yellow glass

IR and NMR spectra were consistent with the indicated structure

Example X:

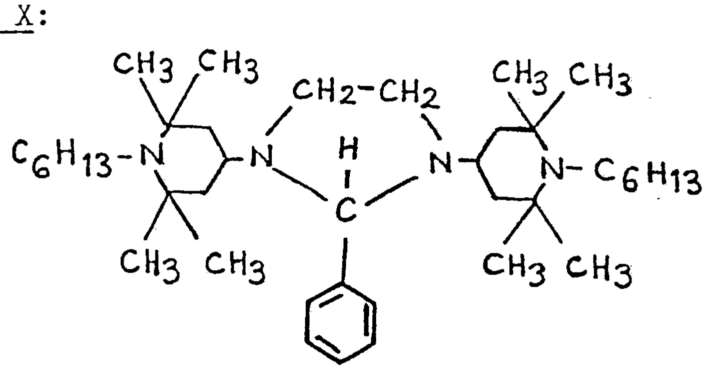

This compound was prepared by the method of Example VI using n-hexyl iodide in place of methyl iodide. The compound was obtained in good yield as a yellow glass. The NMR spectrum was consistent with the above structure.

Example XI:

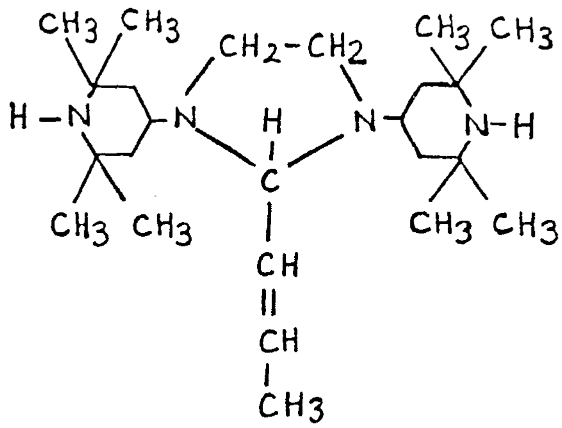

The compound of this example was made by the method of Example I using crotonaldehyde in place of iso-butyraldehyde.

<u>Example XII</u>:

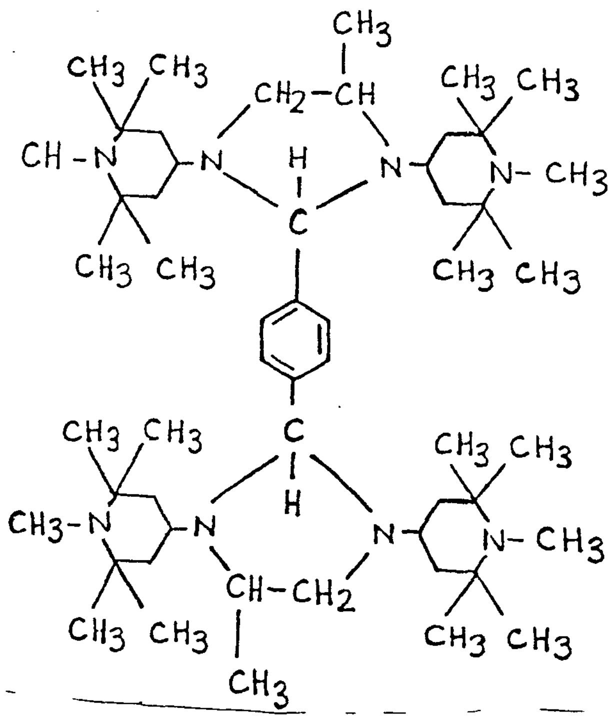

The compound of this example was prepared by the method of Examples IV and VI using the compound of Preparation B in place of the compound of Preparation A.

| | | |
|---|---|---|
| Yield | - | 89.0% |
| MP | - | 211.214°C |
| Colour | - | White |
| NMR | - | Consistent with the above structure. |

<u>Example XIII</u>:

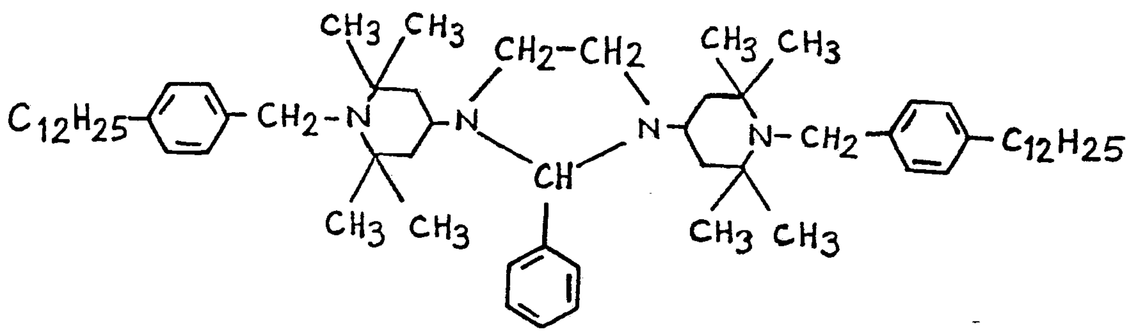

This compound is made by the process of Example VIII wherein dodecyl benzyl chloride is substituted for the benzyl chloride. IR and NMR spectra were consistent with the above structure.

The ability of the hindered amine compounds of this invention to protect organic polymers from degradation by UV light is shown by the following tests.

One hundred parts of polypropylene (Pro-fax 5601 obtainable from Hercules, Inc; Pro-fax is a registered trade mark) was mixed with 0.15 parts of calcium stearate (lubricant), 0.10 parts of tetrakis[methylene (3,5-di-terbutyl-4- hydroxyhydrocinnamate)] methane (antioxidant) and 0.5 parts of various hindered amine compounds of this invention under test (additive). The mixture was extruded and then pelletised. The pellets were then extruded into fibre (20 denier per filament) and tested for tenacity on an Instron Tensile Tester. The filaments were exposed in a Xenon Arc Weather-Ometer and sampled at regular intervals to determine tenacity. The time required to lose 50% of the original tenacity is a measure of UV stability. The results are shown in Table I.

Table 1

POLYPROPYLENE

| Additive | Hours of Exposure for 50% Loss of Tenacity |
|---|---|
| None | 25 |
| Preparation A (Prior Art) | 270 |
| Preparation B (Prior Art) | 255 |
| Example I | 335 |
| Example II | 330 |

| Additive | Hours of Exposure for 50% Loss of Tenacity |
|---|---|
| Example III | 310 |
| Example IV | 320 |
| Example V | 305 |
| Example VI | 300 |
| Example VII | 295 |

Tests with high density polyethylene were performed by mixing 100 parts of polyethylene with 0.05 parts of calcium stearate, 0.03 parts of tetrakis[methylene(3,5-di-terbutyl-4-hydroxyhydrocinnamate)] methane and 0.15 parts of the hindered amine compounds of this invention under test (additive). The mixture was milled on a two-roll mill for 5 minutes at 149°C. The milled samples were compression moulded at 177°C for 6 minutes into 20 mil plaques. The plaques were cut into strips and exposed in a Xenon Arc Weather-Ometer. Samples were removed at regular intervals and tested for degradation by a 180° bending test and the results shown in Table II. The test is terminated after 2 consecutive breaks.

Table II

HIGH DENSITY POLYETHYLENE

| Additive | Hours to Failure (2 consecutive breaks) |
|---|---|
| None | 250 |
| Preparation A(Prior Art) | 2800 |
| Preparation B (Prior Art) | 2800 |
| Example I | 3450 |
| Example II | 3350 |
| Example III | 3300 |
| Example IV | 3400 |
| Example V | 3350 |
| Example VI | 3300 |
| Example VII | 3250 |

The hindered amine compounds of this invention were tested in polyvinyl chloride (PVC) by mixing 100 parts of PVC

Geon 103, (Geon is a registered trademark) with 0.5 parts of dimethyltin bis-isooctyl-thioglycolate, 0.5 parts of stearic acid and 0.2 parts of the hindered amine compound of this invention (additive). After milling on a two-roll mill at 193°C for 5 minutes the resin was pressed into 20 mil plaques and exposed in a Xenon Arc Weather-Ometer. Plaque colours were noted after 400 hours of exposure and the results shown in Table III

Table III

POLYVINYL CHLORIDE

| Additive | Plaque Colour |
|---|---|
| None | Dark Yellow |
| Preparation A (Prior Art) | Yellow |
| Preparation B (Prior Art) | Yellow |
| Example I | Pale Yellow |
| Example II | Pale Yellow |
| Example III | Pale Yellow |
| Example IV | Pale Yellow |
| Example V | Pale Yellow |
| Example VI | Pale Yellow |
| Example VII | Pale Yellow |

Polyurethane compositions, prepared from toluene diisocyanate and alkylene polyols, showed much less yellowing when exposed to sunlight if they were protected by the hindered amine compounds of Examples I, IV, or VI.

# CLAIMS

1. A compound having one or two bis(2,2,6,6-tetramethyl-4-piperidyl) substituted heterocyclic rings in which said heterocyclic ring has five ring atoms, two of which are non-adjacent nitrogen atoms and three of which are carbon atoms, and having a 2,2,6,6-tetramethyl-4-piperidyl group bonded to each nitrogen atom of the heterocyclic ring.

2. A compound according to claim 1 characterised by the following general formula

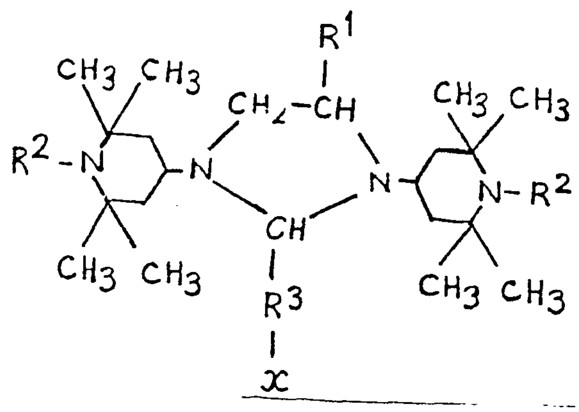

wherein

$R^1$ is hydrogen or methyl,

$R^2$ is hydrogen, alkyl having 1 to 8 carbon atoms, alkenyl having 2 to 8 carbon atoms or aralkyl having 7 to 18 carbon atoms,

$R^3$ is a straight or branched chain alkylene, straight or branched chain alkenylene, unsubstituted or substituted arylene or cycloalkylene group and

X is hydrogen or a group having the general formula

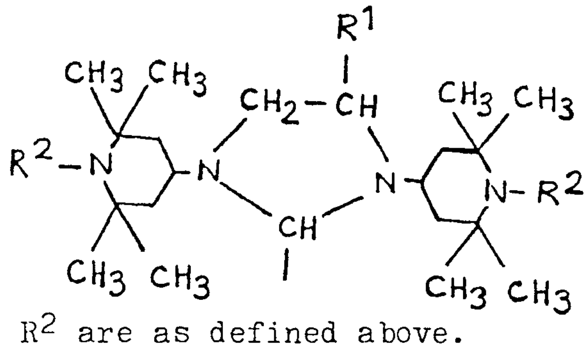

wherein $R^1$ and $R^2$ are as defined above.

3. A compound according to claim 2 further characterised in that $R^3$ is a straight or branched chain alkylene group having from 1 to 14 carbon atoms, a straight or branched chain

alkenylene group having 2 to 14 carbon atoms and 1 to 3 carbon to carbon double bonds, an alkyl substituted or unsubstituted phenylene group or a cycloalkylene group having 5 to 6 carbon atoms in the ring.

4. A compound according to claim 3 further characterised in that $R^1$ is hydrogen or methyl and $R^2$ is hydrogen, a $C_1$ to $C_8$ alkyl group, a $C_2$ to $C_8$ alkenyl group or a $C_7$ to $C_{18}$ aralkyl group.

5. An organic polymer-containing composition characterised in that it comprises an organic polymer normally susceptible to deterioration induced by light, by heat or by both and a stabilisingly effective amount of a compound according to any of claims 1 to 4.

6. A composition according to claim 5 further characterised in that said stabilising amount of said compound is from 0.001% to 10% by weight preferably 0.05 to 3% by weight based on the weight of said organic polymer.

7. A composition according to either of claims 5 or 6 further characterised in that said polymer is a homopolymer of ethylene or propylene, a copolymer of ethylene and propylene or a polymer mixture comprising a homopolymer or copolymer of ethylene or of propylene.

8. A process for making a compound as claimed in any of claims 1 to 4 characterised by reacting a N,N$^1$-bis(2,2,6,6-tetramethyl-4-piperidyl) substituted ethylene diamine or 1,2-propylene diamine with an aliphatic monoaldehyde, or dialdehyde, a cycloaliphatic monoaldehyde or dialdehyde, an aromatic monoaldehyde or dialdehyde, an araliphatic monoaldehyde or dialdehyde, an alkylaromatic monoaldehyde or dialdehyde and, if desired, reacting the product with an alkyl halide, an aralkyl halide or an alkenyl halide.

9. A process according to claim 8 further characterised in that a compound having the general formula

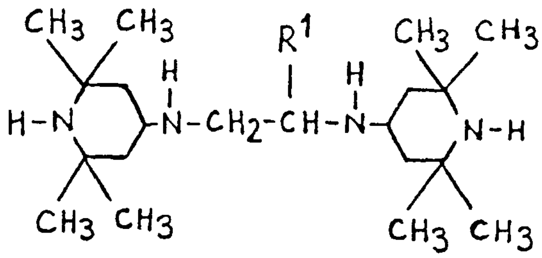

is reacted with an aldehyde having the formula $R^5\left(\overset{O}{\overset{\|}{C}}H\right)_n$ and, if desired, then reacting the product with a halide having the general formula $R^4Y$ wherein

$R^1$ is hydrogen or methyl,

$R^4$ is $C_1$ to $C_8$ alkyl, $C_2$ to $C_8$ alkenyl or $C_7$ to $C_{18}$ aralkyl,

$R^5$ is, when n is 1, a branched or straight chain alkyl group preferably having 1 to 12 carbon atoms, a straight or branched chain alkenyl group preferably having 2 to 12 carbon atoms, a substituted or unsubstituted aryl group or a cycloalkyl group, or when n is 2

$R^5$ is a straight or branched chain alkylene group preferably having 2 to 12 carbon atoms, a straight or branched chain alkenylene group, a substituted or unsubstituted arylene group or a cycloalkylene group,

Y is bromine, chlorine or iodine and

n is 1 or 2.

0033663

European Patent Office

# EUROPEAN SEARCH REPORT

Application number

EP 81300450.4

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| X | BE - A - 877 916 (CHIMOSA) (16-11-1979)<br>+ Claims 1,2,11,15,18,19, 25-28 +<br>& GB-A-2 027 023 (13-02-1980)<br>---- | 1-9 | C 07 D 401/14<br>C 08 K 5/34<br>C 08 L 23/00//<br>(C 07 D 401/14<br>C 07 D 211/58<br>C 07 D 233/02) |
| | | | TECHNICAL FIELDS SEARCHED (Int. Cl.³) |
| | | | C 07 D 401/00<br>C 08 K<br>C 08 L |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 15-04-1981 | HOCHHAUSER |

EPO Form 1503.1 06.78